# EUROPÄISCHE PATENTSCHRIFT

(11) **EP 1 385 567 B1**
(45) Veröffentlichungstag und Bekanntmachung des Hinweises auf die Patenterteilung: **02.11.2011**
(21) Anmeldenummer: 02750908.2
(22) Anmeldetag: 08.05.2002
(51) Int. Cl.: A61M 16/00, A61M 16/04

(54) **Vorrichtung zur Beatmung mit einem Endotrachealtubus**
Device for artificial respiration with an endotracheal tube
Dispositif de respiration artificielle au moyen d'un tube endotracheal

(30) Priorität: 10.05.2001 DE 10123278
(43) Veröffentlichungstag der Anmeldung: 04.02.2004
(73) Patentinhaber: Reissmann, Hajo, 22765 Hamburg (DE)
(72) Erfinder: REISSMANN, Hajo, 22765 Hamburg (DE)
(74) Vertreter: Hauck Patent- und Rechtsanwälte
(86) Internationale Anmeldenummer: PCT/EP2002/005118
(87) Internationale Veröffentlichungsnummer: WO 2002/089885

(56) Entgegenhaltungen:
- EP-A- 0 806 217
- WO-A1-00/45881
- WO-A1-00/45881
- DE-A- 19 528 113
- DE-U1- 8 814 745
- DE-U1- 8 814 745
- GB-A- 2 318 518
- US-A- 3 102 537

## Beschreibung

Die Erfindung bezieht sich auf eine Vorrichtung zur Beatmung mit einem Endotrachealtubus.

Zur Beatmung im Rahmen einer Intensivtherapie oder während einer Narkose werden die Patienten in der überwiegenden Mehrzahl endotracheal intubiert. Dabei wird ein Endotrachealtubus, der in der Regel aus einem Kunststoff- oder einem Gummirohr besteht, durch Mund, Nase oder Luftröhrenschnitt ("Tracheotomie") in die Luftröhre eingeführt. Der Endotrachealtubus ist meist am trachealen Ende mit einer aufblasbaren Tubusmanschette ("Cuff") zur Abdichtung der Luftröhre versehen, die die Beatmung mit Überdruck ermöglicht und die Atemwege vor dem Eindringen von Fremdmaterial schützt. Das patientenferne Ende des Endotrachealtubus ist über ein Schlauchsystem an ein Beatmungsgerät angekoppelt. Dabei befindet sich am Übergang vom Endotrachealtubus zum Schlauchsystem ein Y-Verbinder, an den ein Einatemschlauch und ein Ausatemschlauch oder ein Ausatemventil zur Atmosphäre angeschlossen sind.

Zwischen Endotrachealtubus und Schlauchsystem kann ein Atemluftfilter angeordnet sein, um den Austausch von Mikroorganismen und anderen Verunreinigungen zwischen Patient und Schlauchsystem zu minimieren. Die hierdurch mögliche Verlängerung der Zeitabstände zwischen Schlauchwechseln ist ökonomisch und ökologisch vorteilhaft. Ferner können zwischen Schlauchsystem und Endotrachealtubus Wärme-Feuchte-Tauscher vorhanden sein, die ein Befeuchten der Einatemluft bewirken, um eine Austrocknung der Atemwege des Patienten zu verhindern. Dies geschieht durch Kondensation der in der Ausatemluft enthaltenen Feuchtigkeit an speziellen filterähnlichen Materialien und Wiederverdunstung der Feuchtigkeit bei der folgenden Einatmung. Ferner gibt es die sogenannte "aktive Befeuchtung" durch einen beheizten Verdunster im Einatemschlauch des Schlauchsystems.

Die Lunge betreibt den Gasaustausch mit der Außenluft in einem Pendelverfahren. Daher gelangt bei jeder Einatmung zunächst Gas in die Lunge, das von der vorherigen Ausatmung im sogenannten "Totraum" verblieben ist. Der Totraum ist die Gesamtheit aller Luftleiter, die während sowohl der Ein- als auch der Ausatmung durchströmt werden. Er umfaßt die Bronchien und die Luftröhre ebenso wie den konventionell einlumigen Endotrachealtubus und eventuell vorgeschaltete Atemluftfilter oder Wärme-Feuchte-Tauscher. Er endet in einem Y-Verbinder, der den Luftweg für die Ein- und Ausatmung verzweigt.

Die Atemexkursionen führen zu mechanischen Belastungen der Lunge und erfordern vom spontan atmenden Patienten Atemarbeit. Da der (anatomische und apparative) Totraum einen erheblichen Anteil des Atemhubes ausmachen kann (etwa 30% bis über 50%), ist seine Minimierung ein wichtiger Schritt zur Minderung dieser Belastungen und Atemarbeitsanteile. Eine bekannte Maßnahme zur Totraumminderung besteht in dem Einsatz eines Endotrachealtubus mit zwei Lumina ("doppellumiger Endotrachealtubus"), die separat mit dem Einatemschlauch und dem Ausatemschlauch oder dem Ausatemventil zur Atmosphäre verbunden werden. Ein Beatmungssystem mit einem doppellumigen Endotrachealtubus ist beispielsweise in der DE 25 35 191 Al beschrieben. Bei einem doppellumigen Endotrachealtubus ist die Trennung von Inspirations- und Expirationsschenkel der Beatmungsvorrichtung an die Stelle der Verbindung der beiden Lumina verlegt. Diese befindet sich tief in der Luftröhre am trachealen Ende des Endotrachealtubus. Entsprechend ist der Totraum des Systems und die durch ihn bedingte Atemarbeit verringert.

Ein weiterer Vorteil doppellumiger Endotrachealtuben ist bei einer druckregelnden Beatmung gegeben. Bei einer solchen Beatmung ist der Druck am Y-Verbinder Ziel der Regelung durch das Beatmungsgerät. Dazu verzichten die üblichen Beatmungsgeräte auf einen separat bis zum Y-Verbinder geführten Meßabgriff, denn der dortige Druck läßt sich ebenso gut über die stehende Gassäule des in der jeweiligen Atemphase gerade nicht durchströmten Schlauches messen. Mit einem doppellumigen Endotrachealtubus wird der Y-Verbinder und damit der Regelpunkt in die Luftröhre verlegt. In direkter Konsequenz muß nicht mehr der Patient denjenigen Teil der Atemarbeit überwinden, der von den Strömungswiderständen in einem einlumigen Endotrachealtubus verursacht wird. Die Überwindung der Strömungswiderstände des doppellumigen Endotrachealtubus fällt automatisch dem Beatmungsgerät zu.

Wenn der Endotrachealtubus knickt oder sich Fremdmaterial, etwa Sekret aus der Luftröhre, in seinem Inneren festsetzt, nimmt sein Widerstand zu. Dies kann zur Gefährdung des Patienten durch Behinderungen der Beatmung und/oder Aufbau hoher Drücke in der Lunge (bei Einschränkung vornehmlich der Ausatmung) führen. Da derartige Veränderungen vielfach den einer Sichtkontrolle nicht zugänglichen Teil des Endotrachealtubus treffen, werden erst späte Zeichen, also Minderungen der Ventilation oder Drucksteigerungen, bemerkt.

Diese Nachteile können eingeschränkt durch Überwachen des Druckes am Y-Verbinder und der Gasflüsse überwunden werden. Die mechanischen Eigenschaften der Strukturen abseits des Y-Verbinäers (Filter, Endotrachealtubus, Atemwege, Lunge, umgebendes Gewebe, Muskelaktivität) kann diese Überwachung jedoch nicht separieren und zuordnen. Daher ist eine Überwachung der Tubus-Eigenschaften sehr eingeschränkt möglich, und es werden allenfalls grobe Veränderungen erfaßt.

Ein weiteres Problem liegt darin, daß die Einatemluft des Patienten regelmäßig angefeuchtet werden muß, weil der Endotrachealtubus sie von den natürlichen Befeuchtern (Schleimhäute der Nase und des Rachens) fernhält. Wärme- und Feuchtetauscher fungieren in der Regel gleichzeitig als Filter und damit als Kontaminationsbarriere zwischen Patient und Schlauchsystem. Sie müssen jedoch wechselweise von Ausatem- und Einatemluft durchströmt werden, um ihre Funktion zu erfüllen. Daher sind sie nicht bei doppellumigen Endotrachealtuben einsetzbar. Derzeit ist bei doppellumigen Endotrachealtuben nur eine aktive Befeuchtung mit elektrisch beheizten Verdunstern möglich.

Ein weiteres Problem ist die Entfernung von Sekret und Fremdmaterial aus dem Endotrachealtubus. Während einer Beatmungstherapie kann der natürliche Reinigungsmechanismus der Atemwege Sekret und Fremdmaterial allenfalls in die Luftröhre bis zum Endotrachealtubus befördern. Spätestens dort muß es abgesaugt werden. Hierzu wird üblicherweise der Y-Verbinder vom Endotrachealtubus dekonnektiert, damit ein Absaugkatheter in den Endotrachealtubus eingefiihrt werden kann. Die Beatmung wird dadurch unterbrochen und ein etwaiger kontinuierlich positiver Atemwegsdruck (CPAP) bzw. positiver end-expiratorischer Druck (PEEP) - eine übliche Prophylaxe gegen den Kollaps von erkrankten Lungenanteilen - kann nicht gehalten werden. Daher werden nach dem Absaugen besondere Blähmanöver zum Wiedereröffnen kollabierter Lungenanteile erforderlich.

Des weiteren ist schon bekannt, den Absaugkatheter durch einen speziellen Winkeladapter zwischen Endotrachealtubus und Y-Verbinder einzuführen, der um den Katheter herum eine Dichtung bildet. Das Beatmungsgerät bleibt mit dem Endotrachealtubus konnektiert und die Beatmung kann grundsätzlich fortgesetzt werden. Hierbei ergeben sich allerdings Probleme dadurch, daß - je nach Dimensionierung des Endotrachealtubus und des Absaugkatheters - der für die Beatmung verfügbare Anteil des Lumens des Endotrachealtubus gemindert und damit sein Widerstand für Ein- und Ausatemluft mehr oder weniger stark gesteigert ist. Neuere Studien zeigen, daß die Fortsetzung einer volumenkontrollierten Beatmung unter diesen Bedingungen auf jeden Fall zu vermeiden ist: Je nach Beatmungsmuster und den verschiedenen Schlauchdimensionen sind gefährliche Über- oder Unterdrucke möglich. Wenn daher unter Absaugung die Beatmung fortgesetzt werden soll, muß dies unbedingt in einem druckgeregelten Modus geschehen. Auch dann wird mit konventionellem einlumigen Endotrachealtubus der Druck in der Luftröhre beim Absaugen immer deutlich unter dem eingestellten Zieldruck liegen, denn zwischen Luftröhre und Y-Verbinder, dem Meß- und Regelpunkt, befindet sich der Endotrachealtubus mit seinem reduzierten Lumens.

Die DE 195 28 113 A offenbart eine Beatmungseinrichtung zur kontrollierten, mechanischen Beatmung von Patienten mit einer Messung und Auswertung der exspiratiorischen Zeitkonstante des respiratorischen System. Mittels charakteristischer Veränderungen der Zeitkonstanten-Volumen-Beziehung sollen Widerstands-Erhöhungen bzw. Obstruktionen von Endotrachealtubus einerseits und Luftröhre und Bronchien andererseits erkannt und differenziert werden.

Die GB 2 318 518 offenbart einen Doppellumenendotrachealtubus, bei dem ein Lumen der Zufuhr eines kontinuierlichen Frischgasstromes und das größere Lumen einer unterbrochenen Exspiration dient. Durch phasischen Verschluß des größeren Lumens erfolgt eine kontrollierte Beatmung. An den Exspirationszweig des Gerätes ist ein Drucksensor angeschlossen.

Die US 3 102 537 A offenbart ein Atmungsgerät mit einer Gesichtsmaske, insbesondere für die Luft- und Raumfahrt, das einen verbesserten

Feuchtigkeitstransport von der ausgeatmeten zur eingeatneten Luft und eine maximale Ersparnis an Sauerstoff oder einem anderen Einatmungsgas aus einer externen Quelle gewährleistet. Zwischen dem exspiratorischen und dem inspiratorischen Luftleiter ist eine feuchtigkeitsperrneable, gemeinsam Wand angeordnet. Zusätzlich ist ein Reservoir vorhanden, in das eine erste Porion des Ausatemgases gelangt, um bei der folgenden Inspiration zuerst eingeatmet zu werden.

Davon ausgehend liegt der Erfindung die Aufgabe zugrunde, eine knkfionell verbesserte Vorrichtung zur Beatmung mit einem Endotrachealtubus zu schaffen.

Diese Aufgabe wird durch eine Vorrichtung zur Beatmung mit den Merkmalen des Anspruchs 1 gelöst.

Die Erfindung basiert darauf, daß es möglich ist, aufgrund der Meßwerte des Gasstromes in dem durchströmten Lumen und der Drucke an den beiden Lumen den Strömungswiderstand des durchströmten Lumens zu ermitteln. Der mit dem patientenfernen Ende des durchströmten Lumens verbundene Druckmesser mißt nämlich direkt den Druck am patientenfernen Ende dieses Lumens, und der mit dem patientenfernen Ende des nicht durchströmten Lumens verbundene Druckmesser mißt den Druck am trachealen Ende des durchströmten Lumens, so daß sich aus den beiden Drucken der Druckverlust des durchströmten Lumens ergibt. Zusammen mit dem Gasdurchfluß des durchströmten Lumens läßt sich dann der Strömungswiderstand des durchströmten Lumens errechnen. Hierzu wird auf die aus der Strömungslehre bekannten Zusammenhänge zwischen Druckverlust, Volumenstrom und Widerstandszahl durchströmter Rohre Bezug genommen. Aus dem Strömungswiderstand läßt sich wiederum direkt darauf zurückschließen, ob der Endotrachealtubus unzulässig geknickt oder verstopft ist, so daß Gegenmaßnahmen ergriffen werden müssen. Bei einer softwaregesteuerten Vorrichtung zur Beatmung können die beschriebenen Auswerte- und Ausgabevorgänge leicht softwaregesteuert verwirklicht werden.

Für sämtliche erfindungsgemäßen Vorrichtungen zur Beatmung gilt, daß das Lumen für die Ausatmung über ein Ventil - vorzugsweise ein aktiv gesteuertes Ventil - mit der Atmosphäre verbunden sein kann oder über einen Ausatemschlauch mit einem Eingang des Beatmungsgerätes für ausgeatmete Atemluft verbunden sein kann. Ersteres kann insbesondere bei Notfall-Beatmungsgeräten der Fall sein. Die Rückführung der Ausatmung in das Beatmungsgerät ist insbesondere bei der Narkosebeatmung üblich, wenn verhältnismäßig teure Gase der Beatmung zugeführt werden und eine Rückgewinnung der Gase aus der Ausatmung vorteilhaft ist.

Die Durchflußmesser und/oder die Druckmesser können am Beatmungsgerät und/oder am Ende des Trachealtubus angeordnet sein. Vorzugsweise befinden sie sich am Beatmungsgerät, da durch die Anordnung am Ende des Trachealtubus dessen Handhabbarkeit erschwert wird. Falls die Ausatemseite des Endotrachealtubus nicht mit dem Beatmungsgerät über einen Ausatemschlauch verbunden ist, ist die Anordnung des Durchflußmessers und des Druckmessers der Ausatemseite am Endotrachealtubus obligatorisch. Auch in diesem Falle sind jedoch bevorzugt der Durchflußmesser und der Druckmesser der Einatemseite im Beatmungsgerät angeordnet.

Grundsätzlich ermittelt die Vorrichtung den Strömungswiderstand des durchströmten Lumens und des damit verbundenen Schlauches zum Beatmungsgerät. Hat man der Vorrichtung die Eigenschaften des Schlauches ohne Endotracheltubus bekanntgemacht, kann sie den Strömungswiderstand des Endotrachealtubus ermitteln. Zum Ermitteln der Eigenschaften des Schlauches kann dieser vom Endotrachealtubus dekonnektiert und zur Umgebung geöffnet oder können zwei Schläuche miteinander verbunden und zu Testzwecken mit Gasströmen durchströmt werden. Falls der Schlauch zur Umgebung geöffnet ist, kann der Druckverlust mit Hilfe des bekannten Umgebungsdruckes und dem an einem Schlauchende gemessenen Druck und dem gemessenen Gasstrom ermittelt werden. Bei Verbindung der Schläuche miteinander gehen die an den Enden der Schläuche gemessenen Drucke in die Berechnung ein. Vorzugsweise sind zwischen dem Endotrachealtubus und dem Schlauch/den Schläuchen Ventileinrichtungen vorhanden, um den Schlauch/die Schläuche mit Umgebung und/oder miteinander zu verbinden.

Ferner wird ein Beispiel einer Vorrichtung zur Beatmung offenbart. Diese hat
- einen Endotrachealtubus und
- mindestens eine wasserdurchlässige Membran in einer Wand zwischen zwei Lumen des Endotrachealtubus, die Wasser bevorzugt von der mit einem ausgeatmeten Gasstrom beaufschlagten Seite zu der mit einem Gasstrom für die Beatmung beaufschlagten Seite leitet.

Auf der Seite der wasserdurchlässigen Membran, die dem Ausatemzweig angehört, schlägt sich die Feuchtigkeit aus der Ausatemluft nieder, um auf der anderen Seite der Membran, die zum Einatemzweig gehört, von der trockenen Einatemluft wieder aufgenommen zu werden. Da der Feuchtigkeitsaustausch im Gegenstrom arbeitet, kann bei geeigneter Dimensionierung eine hohe Wirksamkeit erreicht werden. Diese kann durch Beheizen des Gasstromes im Einatemzweig gesteigert werden, da warme Einatemluft die Feuchtigkeit schneller und besser aufnehmen kann. Ebenso ist eine Steigerung der Wirksamkeit durch Einsatz eines Membranmaterials möglich, das Wasser bevorzugt vom Ausatemzweig zum Einatemzweig transportiert. Derartige "asymmetrische" Materialien sind beispielsweise für den Einsatz in Hygieneprodukten (Säuglingswindeln oder Monatsbinden) bekannt. Eine große Austauschfläche kann ferner dadurch erreicht werden, daß die Schläuche und/oder die Lumen eines doppellumigen Endotrachealtubus einander umhüllen, beispielsweise indem sie einander konzentrisch umgeben.

Die zweite Lösung kann vorteilhaft auch mit der ersten Lösung kombiniert werden. Dies gilt auch für beliebige Ausgestaltungen der ersten und der zweiten Lösung.

Gemäß einer weiteren Ausgestaltung hat die Vorrichtung zur Beatmung
- eine schließbare Öffnung am patientenfernen Ende des Lumens für den ausgeatmeten Gasstrom zum abdichtenden Einsetzen eines Absaugkatheters bis zum trachealen Ende des Endotrachealtubus und
- eine Einrichtung zum Regeln des Druckes in dem nicht von dem Gas durchströmten Lumen auf einen vorgegebenen Wert.

Die druckgeregelte Beatmung über den doppellumigen Endotrachealtubus kann unter Absaugung problemlos fortgesetzt werden, ohne daß es zu Über- oder Unterdrucken kommt, denn der Beatmungsdruck wird genau dort geregelt, wo auch die Absaugung wirksam wird, nämlich in der Luftröhre. Die Einrichtung zum Regeln des Druckes ist mit den Druckmessern verbunden und steuert den Gasstrom. Ferner kann der Beatmungsdruck durch ein Ventil am patientenfernen Ende des Lumens für die Ausatmung gesteuert werden. Voraussetzung für die Steuerung des Druckes über den Gasstrom ist, daß der Einatemschenkel der Vorrichtung, d.h. der Schlauch und das Lumen, über den/das der Gasstrom für die Beatmung zugeführt wird, nicht einen so großen Strömungswiderstand haben, daß das Beatmungsgerät den gewünschten Druck in der Luftröhre nicht aufbauen kann. Durch das Einsetzen des Absaugkatheters in das Lumen, durch das die Ausatemluft abgeführt wird, wird eine nachteilige Erhöhung des Strömungswiderstandes in dem anderen Lumen vermieden. Bevorzugt wird zusätzlich das Lumen für den ausgeatmeten Gasstrom mit einem größeren Querschnitt ausgeführt, als das Lumen für den Gasstrom zur Beatmung (asymmetrischer doppellumiger Endotrachealtubus). Durch die Anordnung des Absaugkatheters könnte eine Einschränkung der Ausatmung resultieren; dieser Effekt wird jedoch durch den Gasstrom der Absaugung gemildert oder aufgehoben, der auch eine Art von Ausatmung (am Beatmungsgerät vorbei) bewirkt. In jedem Falle wird der vom Anwender vorgegebene Druckbereich zu keinem Zeitpunkt verlassen. Damit wird es erstmals möglich, eine Beatmungstherapie durchzuführen, bei der zu keinem Zeitpunkt, auch nicht im Zusammenhang mit Absaugmanövern, der vorgegebene CPAP bzw. PEEP (s.o.) unterschritten wird.

Gemäß einer weiteren Ausgestaltung hat die Vorrichtung zur Beatmung
- eine Einrichtung zum Aufbringen eines hochfrequent oszillierenden Druckes auf das patientenferne Ende des mit dem Einatemschlauch verbundenen Lumens des Endotrachealtubus für das einzuatmende Gas.

Durch das Aufbringen einer hochfrequenten Druckschwankung auf den Einatemschenkel der Beatmungsvorrichtung wird der Gasaustausch mit dem Patienten verbessert. Der Gasstrom zur Beatmung des Patienten kann hierdurch reduziert werden. Unter "hochfrequent" werden hierbei Frequenzen verstanden, die die Atemfrequenz des Patienten übersteigen, insbesondere Frequenzen, die über etwa mindestens 5 Hz betragen. Das Aufbringen einer hochfrequenten Druckschwingung ist zwar schon in Verbindung mit eintumigen Endotracheahuben bekannt. Hier müssen jedoch wegen des erheblichen Totvolurnens Druckschwankungen hoher Energie aufgebraucht werden, um eine Verbesserung des Gasaustausches zu erreichen. Die Bereitstellung geeigneter Oszillatoren ist problematisch. In Verbindung mit einem doppellurrigen Endotrachealtubus wird wegen des stark verminderten Totvolumens die Verbesserung des Gasaustausches bereits mit Oszillatoren erreicht, die verhältnisnmäßig geringe Energien und Amplituden aufweisen. Die Ausbreitung der Druckschwankungen in die Lunge kann durch die Ausbildung des Lumens für die. Ausatmung als Tiefpaß für die Druckschwingung gefördert werden. Vorzugsweise ist im Ausatemschenkel der Beatmungsvorrichtung ein aktives Ventil vorhanden, dessen Betätigung mit der OsziUation des Druckerzeugers koordiniert wird.

Die Erfindung wird nachfolgend anhand der anliegenden Zeichnung erläutert, die grob schematisch ein Ausfuhrungsbeispiel zeigt.

Die Vorrichtung zur Beatmung umfaßt ein Beatmungsgerät 1 mit einem Ausgang 2, an dem ein Gasstrom zur Beatmung bereitgestellt wird, und einem Eingang 3, dem ein ausgeatmeter Gasstrom zugeführt werden kann. Das Beatmungsgerät 1 hat im Eingang 3 ein aktives Ventil, das beim Einatmen geschlossen wird, um den Druck in der Lunge zu halten, und das beim Ausatmen geöffnet wird. Hinter dem Ventil kann das Ausatmungsgas der Umgebung zugeführt werden. Statt dessen kann im Beatmungsgerät 1 der ausgeatmete Gasstrom aufgearbeitet (CO₂ wird entfernt) und das für die Beatmung benötigte Gas wird erneut dem Ausgang zugeführt werden, wobei frisches Beatmungsgas zugemischt werden kann.

Ferner ist ein Endotrachealtubus 4 vorhanden, der zwei Lumen 5, 6 aufweist. Am trachealen Ende hat dieser doppellumige Endotrachealtubus 4 eine aufblasbare Manschette 7, die zu einer Luftröhre 8 hin abdichtet, wobei der Endotrachealtubus 4 bis kurz vor die Bronchien 9 vorgeschoben ist.

Das Beatmungsgerät 1 ist über ein Schlauchsystem 10 mit dem patientenfernen Ende des Endotrachealtubus 4 verbunden. Das Schlauchsystem 10 hat einen Einatmungsschlauch 11, der mit dem Ausgang 2 und dem Lumen 5 verbunden ist. Ferner hat es einen Ausatmungsschlauch 12, der mit dem Lumen 6 und dem Eingang 3 verbunden ist.

Im Beatmungsgerät 1 befinden sich Durchflußmesser zum Messen der Gasströme aus dem Ausgang 2 und in den Eingang 3. Ferner befinden sich im Beatmungsgerät 1 Druckmesser zum Messen des Druckes im Ausgang 2 und im Eingang 3.

Mit Hilfe der gemessenen Durchfluß- und Druckwerte rechnet ein in das Beatmungsgerät integrierter Rechner den Strömungswiderstand in dem gerade durchströmten Lumen 5 bzw. 6. Bei Überschreitung unzulässiger Werte wird auf ein Knicken bzw. Verstopfen des Endotrachealtubus 4 geschlossen und ein optisches und/oder akustisches Signal ausgegeben.

Ferner ist die Wand zwischen den beiden Lumen 5, 6 in einem Bereich als wasserdurchlässige Membran 13 ausgebildet. Durch diese Membran 13 wird Feuchtigkeit von der Ausatemluft im Lumen 6 auf die Einatemluft im Lumen 5 übertragen.

Ferner ist das Lumen 6 durch eine Öffnung 14 verschlossen, durch die im Bedarfsfalle ein Absaugkatheter abdichtend einführbar ist bis zum trachealen Ende des Endotrachealtubus 4. Durch Messung des Druckes am Ausgang 2 und Eingang 3 und Regelung der Gasströme des Beatmungsgeräts 1 wird erreicht, daß der Druck in der Luftröhre auf einem konstanten Wert gehalten wird.

Schließlich kann im Beatmungsgerät 1 eine Einrichtung zur Erzeugung eines pulsierenden Druckes mit einer die Atemfrequenz übersteigenden Frequenz angeordnet sein, die auf den Ausgang 2 wirkt, um den Gasaustausch bei der Beatmung zu intensivieren.

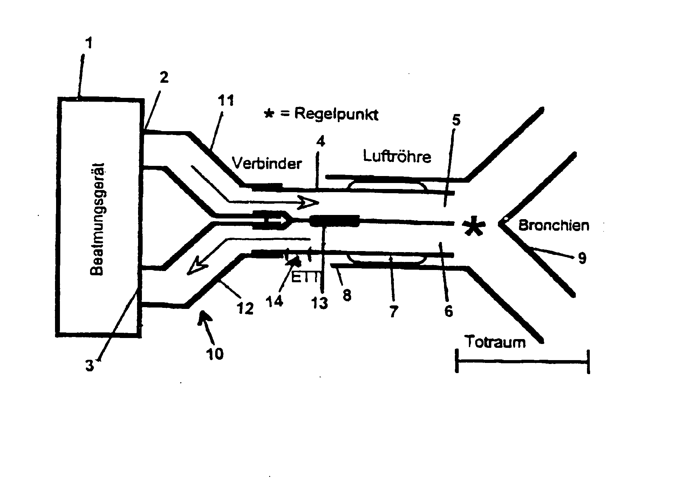

## Patentansprüche

1. Vorrichtung zur Beatmung mit
- einem Beatmungsgerät (1) zum Bereitstellen eines Gasstromes zur Beatmung an einem Ausgang (2),
- einem Einatemschiauch (11), der einenends mit dem Ausgang (2) verbunden ist,
- einem doppellumigen Endotrachealtubus (4), von dem das eine Lumen (5) an seinem patientenfernen Ende mit dem anderen Ende des Einatemschlauchs (11) verbunden ist,
- Durchflussmessern zum Messen der Gasströme in den beiden Lumen (5, 6) des Endotrachealtubus (4),
- mit den patientenfernen Enden der beiden Lumen (5, 6) verbundenen Dmckmessern zum Messen der Drucke an den patientenfernen Enden der beiden Lumen (5, 6),
- einer Auswerteeinrichtung zum Ermitteln des Strömungswiderstandes in dem gerade durchströmten Lumen (5, 6) aufgrund des darin gemessenen Gasstromes und der gemessenen Drucke an den patientenfernen Enden der beiden Lumen (5,6) und
- einer Einrichtung zum Ausgeben einer Information über den Strömungswiderstand der Lumen (5, 6).

2. Vorrichtung nach Anspruch 1, bei der die Durchflussmesser und/oder die Drucksensoren in das Beatmungsgerät (1) integriert ist/sind.

3. Vorrichtung nach Anspruch 1 oder 2, bei der zwischen dem Schlauch/den Schläuchen und dem Endotrachealtubus (4) Vcntileinrichtungen des Schlauches/der Schläuche mit der Umgebung und/oder miteinander vorhanden sind.

4. Vorrichtung nach einem der Ansprüche 1 bis 3, bei der die Durchflussmesser und/oder die Drucksensoren in das Beatmungsgerät (1) integriert ist/sind.

5. Vorrichtung nach einem der Ansprüche 1 bis 4, bei der zwischen dem Schlauch/den Schläuchen und dem Endotrachealtubus (4) Ventileinrichtungen des Schlauches/der Schläuche mit der Umgebung und/oder miteinander vorhanden sind.

6. Vorrichtung zur Beatmung nach einem der Ansprüche 1 bis 5, mit
- einer schließbaren Öffnung am patiemenfernen Ende des Lumens für den ausgeatmeten Gasstrom zum abdichtenden Einsetzen eines Absaugkatheters bis zum trachealen Ende des Endotrachealtubus (4) und
- einer Einrichtung zum Regeln des Druckes in dem nicht von dem Gas durchströmten Lumen (6).

7. Vorrichtung zur Beatmung nach einem der Ansprüche 1 bis 6, mit
- einer Einrichtung zum Aufbringen eines hochfrequent oszillierenden Druckes auf das patientenfernc Ende des Lumens (5) des Endotrachealtubus (4) für das einzuatmende Gas.

## Claims

1. A device for ventilation, comprising
- a ventilator (1) for providing a stream of gas for ventilation at an outlet (2),
- a hose for inspiration air (11) one end of which is connected to the outlet (2),
- a double-lumen endotracheal tube (4) one lumen (5) of which, at its end distal to the patient, is connected to the other end of the hose for inspiration air (11),
- flow meters for measuring the streams of gas in the two lumina (5, 6) of the endotracheal tube (4),
- pressometers connected to the ends distal to the patient of the two lumina (5, 6) for measuring the pressures at the ends distal to the patient of the two lumina (5, 6),
- an evaluation means for determining the flow resistance in a lumen (5, 6) flowed through by gas because of the stream of gas measured therein and the pressures measured at the ends distal to the patient of the two lumina (5, 6), and
- a means for outputting an information about the flow resistance of the lumina (5, 6).

2. The device according to claim 1 wherein the flow meters and/or pressure sensors is/are integrated in the ventilator (1).

3. The device according to claim 1 or 2 wherein valve means of the hose/hoses are connected with the environment and/or with each other between the hose/hoses and the endotracheal tube (4).

4. The device according to one of the claims 1 to 3 wherein the flow meters and/or pressure sensors is/are integrated in the ventilator (1).

5. The device according to one of the claims 1 to 4 wherein valve means of the hose/hoses are connected with the environment and/or with each other between the hose/hoses and the endotracheal tube (4).

6. Device for ventilation according to one of the claims 1 to 5, comprising
- a closable opening at the end distal to the patient of the lumen for the stream of expired gas to sealingly introduce a suction catheter up to the tracheal end of the endotracheal tube (4), and
- a means for regulating the pressure in the lumen (6) which is not flowed through by gas.

7. Device for ventilation according to one of the claims 1 to 6, comprising
- a means for applying a high-frequency oscillating pressure to the end distal to the patient of the lumen (5) of the endotracheal tube (4) for the gas to be inspired.

## Revendications

1. Dispositif de respiration artificielle comprenant
- un appareil de respiration artificielle (1) pour la mise à disposition d'un courant gazeux pour la respiration artificielle à une sortie (2),
- un tuyau d'inspiration (11) qui est relié à une extrémité à la sortie (2),
- un tube endotrachéal à double lumière (4) duquel une lumière (5) est reliée à son extrémité éloignée du patient à l'autre extrémité du tuyau d'inspiration (11),
- des débitmètres pour mesurer les courant gazeux dans les deux lumières (5, 6) du tube endotrachéal (4),
- des manomètres reliés aux extrémités éloignées du patient des deux lumières (5, 6) pour mesurer les pressions aux extrémités éloignées du patient des deux lumières (5, 6),
- un dispositif d'évaluation pour déterminer la résistance au courant dans la lumière parcourue de manière droite (5, 6) en raison du courant gazeux mesuré en son sein et des pressions mesurées aux extrémités éloignées du patient des deux lumières (5, 6) et
- un dispositif pour émettre une information concernant la résistance au courant des lumières (5, 6).

2. Dispositif selon la revendication 1, dans lequel les débitmètres et/ou les capteurs de pression sont intégrés dans l'appareil de respiration artificielle (1).

3. Dispositif selon la revendication 1 ou 2, dans lequel des dispositifs de clapet du tuyau/des tuyaux avec l'environnement et/ou entre eux sont présents entre le tuyau/les tuyaux et le tube endotrachéal (4).

4. Dispositif selon l'une quelconque des revendications 1 à 3, dans lequel les débitmètres et/ou les capteurs de pression sont intégrés dans l'appareil de respiration artificielle (1).

5. Dispositif selon l'une quelconque des revendications 1 à 4, dans lequel des dispositifs de clapet du tuyau/des tuyaux avec l'environnement et/ou entre eux sont présents entre le tuyau/les tuyaux et le tube endotrachéal (4).

6. Dispositif de respiration artificielle selon l'une quelconque des revendications 1 à 5, comprenant
- une ouverture pouvant être fermée à l'extrémité éloignée du patient de la lumière pour le courant gazeux expiré en vue de l'insertion étanche d'un cathéter d'aspiration jusqu'à l'extrémité trachéale du tube endotrachéal (4) et
- un dispositif pour réguler la pression dans la lumière (6) qui n'est pas parcourue par le gaz.

7. Dispositif de respiration artificielle selon l'une quelconque des revendications 1 à 6, comprenant
- un dispositif pour fournir une pression oscillante à haute fréquence à l'extrémité éloignée du patient de la lumière (5) du tube endotrachéal (4) pour le gaz à inspirer.
